# EUROPEAN PATENT SPECIFICATION

(11) **EP 4 467 635 B1**
(45) Date of publication and mention of the grant of the patent: **01.04.2026**
(21) Application number: 24000053.9
(22) Date of filing: 08.05.2024
(51) Int. Cl.: C12M 1/32, B01L 3/00, C12M 1/00, C12M 1/12

(54) **A COVER FOR A MICROTITER PLATE, AS WELL AS A METHOD FOR GROWING CELLS IN A MICROTITER PLATE USING SAID COVER**
ABDECKUNG FÜR EINE MIKROTITERPLATTE SOWIE VERFAHREN ZUM ZÜCHTEN VON ZELLEN IN EINER MIKROTITERPLATTE UNTER VERWENDUNG DER ABDECKUNG
COUVERCLE POUR PLAQUE DE MICROTITRAGE, ET PROCÉDÉ DE CULTURE DE CELLULES DANS UNE PLAQUE DE MICROTITRAGE UTILISANT LEDIT COUVERCLE

(30) Priority: 24.05.2023 NL 1044616; 23.06.2023 NL 1044626
(43) Date of publication of application: 27.11.2024
(73) Proprietor: Enzyscreen B.V., 2105 MB Heemstede (NL)
(72) Inventor: Wouter, Adriaan Duetz, 2105 MB Heemstede (NL)
(74) Representative: de Hoog, Johannes Hendrik

(56) References cited:
- WO-A1-2022/236146
- US-A1- 2016 250 632
- US-A1- 2023 001 410

## Description

The present invention relates to a cover for a microtiter plate,
said microtiter plate comprising a multitude of square wells, each well being defined by a bottom and upright walls chosen from partition walls adjacent to other wells and non-partition walls, wherein the cover comprises
- a lid comprising a rectangular, flat main body,
- at the circumference of the cover members that together with the flat main body define a space for receiving a top side of the microtiter plate,
- a rectangular elastomeric body, and
- a microporous filter in said space sandwiched between the main body and the elastomeric body; wherein the lid and the elastomeric body comprise through-holes for gas exchange between the wells and the surrounding atmosphere via the microporous filter.

Microtiter plates (MTPs) are used to grow prokaryotic cells or more typically eukaryotic cells such as HEK-cells, CHO-cells etc.. The MTPs comprise an array of wells, typically 24 or 96, each well comprising a bottom and upright walls which together define a lumen in which a liquid cell growth medium is provided. At the top of a MTP the wells are open. While growing cells in a microtiter plate, the MTP is provided with a cover. A state of the art cover comprises a flat, rubber mat that is placed on top of the MTP. Said mat is provided in a metal lid comprising a flat main body with at its circumference downward extending flanges.

In use, a top section of the MTP is received in the metal lid. The objective of the cover is to seal the rubber mat against the circumferential upper edge of the wells as defined by the upright walls. This is to avoid contamination of the content of a well with cells from an adjacent well while the MTP is shaken (e.g. with 240 rpm) in an incubator or on an orbital shaker. To allow for the exchange of gases (in particular oxygen to the cells and removal of excess CO₂ generated by the cells), both the rubber mat and the metal lid are provided with an array of through-holes corresponding to the array of wells. To prevent contamination from the surrounding atmosphere through these through-holes, e.g. by bacteria or fungi, a submicron porous filter is present between the rubber mat and flat main body of the metal lid, said filter extending over all the through-holes of the rubber mat. A covers composed of a lid, elastomeric body and a filter is generally referred to as a sandwich cover.

To grow the cells, the MTPs provided with covers are placed on the platform of an orbital shaker. The combined mass of a plurality of MTPs provided with covers on a shaker typically exceeds a kilo or more. This has several adverse effects, amongst which vibrations which may have an effect on growth and/or increase the risk of cross-contamination between adjacent wells in case of aerosol droplet formation.

The object of the present invention is to provide a cover that allows for growing cells in a standardized microtiter plate with reduced vibrations while ensuring that cross-contamination of the content of adjacent wells is avoided.

To this end, the rectangular, flat main body is a plate; and the elastomeric body comprises a grid of grooves for receiving an upper portion of the upright walls of the microtiter plate, grooves of the grid comprising at least one of
- a first longitudinally extending groove wall section at a first angle between 20° and 75° with respect to a main plane of the flat main body, and
- a second longitudinally extending groove wall section at an angle between 160° and 105°;

wherein the groove wall sections are located for contacting said upper portion of the microtiter plate, with a non-partition wall being in contact with a groove wall section chosen from the first and the second groove wall section and a partition wall being in contact with both a first and a second groove wall section of a groove; and
wherein the elastomeric body has a Shore A hardness between 20 and 90.

Thus in use the upper portion of the upright walls of the MTP are received in the grooves grid, wherein edges (ribs) - over their length - of all the upright walls of the MTP defining the wells engage the elastomeric material of the elastomeric body. This provides a very good seal, thus protecting against cross-contamination as it impedes transfer of liquid and/or aerosol from the lumen of one well to the lumen of an adjacent well. Thus for a given weight of the cover better sealing is achieved without having to rely on more clamping a heavier cover, which affects the balance on a shaker; or an equal sealing can be achieved with a lighter cover/with less clamping; or a combination thereof. No longer needing clamps, or being able to use weaker clamps which can have a reduced weight, means that the weight carried by the shaker is reduced relative to the moving mass (shaker table plus any counter weight). This may reduce imbalance and hence undesirable vibrations.

The first and second angle are where an edge of an upright wall contacts the groove wall section without force (as force may deform the elastomeric material). The angles are in a plane perpendicular to the longitudinal direction of the groove, said plane being perpendicular to the flat (which is maintained by being rigid) main body. A groove for receiving a partition wall has two opposite wall cover sections, where the first and second angle define a taper for receiving the upper section of said partition wall. A groove that receives a non-partition wall will have only one wall section chosen from the first wall section and second wall section, said wall section facing away from a centerline of the cover running parallel with said groove. It is preferred that the first angle is between 40° and 70° and the second angle is between 140° and 110°; and more preferably the first angle is between 50° and 65° and the second angle is between 130° and 115°. The cross-section of a groove may be round, or V-shaped, such as flat-bottomed V-shaped.

The cover allows for the use of a lighter lid as its weight is of less relevance to obtain proper sealing of the upright walls against the elastomeric body. In particular, the cover may be used without a clamp which is very suitable for automation of the growth procedure using a robotic system.

In addition or alternatively, the cover allows for the use of lighter clamps to clamp the covered MTPs to the shaker. Clamps are typically used in case of stacks comprising two or more covered MTPs.

The sandwich cover of the present invention is typically a cover for covering standard-sized MTPs and the grid of the elastomeric body will typically be a grid for receiving the walls of an MTP with a standard array of wells, such as 6 wells and more often 24 wells.

The elastomeric body has a Shore A hardness preferably between 25 and 65, more preferably between 40 and 60.

It is preferred that the cover can be released from a MTP without the cover clamping (sticking) to the MTP, which simplifies automated cover removal. To that end, in case the first angle is relatively big and second angle is relatively low (e.g. 65° and 115° respectively; i.e. the taper of the groove is relatively sharp), it is preferred to use a relatively high Shore A hardness, whereas with the first angle being relatively small and the second angle being relatively big (e.g. 45° and 135° respectively), a relatively low Shore A hardness is permissible. Where MTPs with covers are going to be clamped to a shaker table with a clamp, a relatively high Shore A hardness may be chosen.

US20160250632 discloses a cover for multi-well plates. At the inside, the cover may have cylindrical fittings with concave distal ends. The concave distal ends serve to guide the cover when it is placed on a round-well multi-well plate. The ribs of the walls of the wells do not contact these concave distal ends for improved sealing, nor would cylindrical fittings of the cover achieve proper sealing if used for a plate with square wells as with a cover according to the present invention.

WO2022236146 discloses a cover for a multi-well plate where walls of well of an MTP seal against a sterile layer . A flat resilient layer 13 may be present that may be penetrated with a needle).

According to a preferred embodiment, the elastomeric body comprises protrusions at a side of the elastomeric body facing the lid and the main body of the lid comprises undersized holes for receiving the protrusions, and the elastomeric body is attached to the main body with the protrusions received in the undersized holes of the main body.

Such a cover will typically weigh less compared to a cover comprising means for holding the elastomeric body (e.g. metal pins according to a state of the art cover) because of the material saved (undersized holes) and the relatively low density of elastomeric materials.

Such a cover can be made and the elastomeric body held flat (and thus keep the grid of grooves flat) irrespective of the material of the flat main body (plate).

According to a preferred embodiment, the elastomeric body comprises further protrusions at a side of the elastomeric body facing the lid and the flat main body of the lid comprises holes for receiving the further protrusions, and with the elastomeric body attached to the main body with the protrusions received in the undersized holes in the main body the further protrusions protrude from the flat main body.

These further protrusions may serve as distance holders in case covered MTPs are stacked. The protrusions of previous embodiment may also serve this purpose.

According to a preferred embodiment, the members at the circumference of the cover comprise a metal strip comprising a strip body with a multitude of tabs, said tabs extending through slots in the elastomeric body with the distal end of the tabs transverse to the strip body.

Thus in this embodiment, the members are held by the elastomeric body. Such a cover can be manufactured cheaply, even in case of the use of a carbon plate as the flat main body of the lid. To manufacture the cover according to the present invention, the tabs are provided with one or more slots extending parallel to the length of the strip body. Thus the tab is locally weakened and can be bent transverse to the strip body after having been passed through the slots in the elastomeric body. The slots in the elastomeric body are provided close to its circumference and parallel therewith; and extend transverse to the flat main plane of the lid.

While made of metal, the members can be thinner than in conventional covers where they have the same thickness as the flat main body of the lid for the sake of convenience of manufacturing and to provide the state of the art cover with weight to achieve proper sealing. The metal will typically have a thickness of 0.2 mm to 1 mm, preferably between 0.25 mm and 0.5 mm. According to a preferred embodiment, the grooves of the grid comprise at least one of
- a third longitudinally extending groove wall section at a third angle between 70° and 90° with respect to a main plane of the flat main body and with the first longitudinally extending groove wall section between a bottom of the groove and the third longitudinally extending groove wall section, and
- a fourth longitudinally extending groove wall section at a fourth angle between 90° and 110° with respect to a main plane of the flat main body and with the third longitudinally extending groove wall section between a bottom of the groove and the fourth longitudinally extending groove wall section.

This allows for further insurance that contamination between adjacent wells is avoided. Depending on the type of plate, the distance between opposite third and fourth longitudinally extending groove wall sections is chosen such that there the elastomeric body doesn't clamp to MTP, allowing easily automated removal of the cover, such as by a robot. Thus, the groove should be wider than the thickness of the upright wall at the height of the third and fourth longitudinally extending groove wall sections.

According to a practical embodiment, the flat main body is a resilient metal plate.

In contrast to a cover according to the prior art made by bending four sections to form the members surrounding a metal flat main body, the flat main body is not subjected to forces that could possibly compromise the flatness of the flat main body, whether it is due to bending or subsequent welding of corners. The resilient metal plate is preferably made of spring steel. The thickness of the metal plate is preferably between 0.5 mm and 2.5 mm; preferably between 0.7 mm and 1.5 mm.

According to a preferred embodiment, the flat main body is a carbonfiber-resin composite plate. Thus proper sealing of the elastomeric body (which is deformable) against the MTP can be ensured without having to rely on the mass of the lid, as known for metal lids. For shakers that are load-limited, the use of covers according to this embodiment allows for (higher) stacking of covered MTPs when growing cells on a shaker.

The thickness of the carbonfiber-resin composite plate is typically 2 mm or less, preferably 1 mm or less, such as 0.6 mm or less. Typically the minimum thickness will be 0.2 mm and preferably at least 0.4 mm.

Typically the carbonfiber-resin composite is a carbon fiber-epoxy composite.

According to a preferred embodiment, at least one of the flat main body and the elastomeric body comprises at least one recess for the filter.

This allows for positioning the at least filter and/or reducing compression forces exerted on said filter. In case of multiple recesses for a filter in a body, the number of filters will be equal to the number of recesses. For the sake of convenience of manufacturing the cover, typically it will be preferred if the elastomeric body is provided with the at least one recess.

According to a preferred embodiment thereof, the at least one recess for a filter extends from one side of the cover to an opposite side of said side.

This facilitates manufacturing and/or replacement of a filter, as it can be inserted into the recess without disassembly of the cover.

According to a preferred embodiment, at least one of the flat main body and the elastomeric body comprises at plurality of adhesive wells for an adhesive providing a bond between the flat main body and the elastomeric body, said adhesive wells not facing a recess for the filter.

Such a cover can be conveniently manufactured. The adhesive is advantageously chosen to be compatible with heat sterilization (such as at temperature of at least 110°C, preferably at least 120°C).

The invention also relates to a method of growing cells in a microtiter plate comprising square wells, wherein wells of the microtiter plate are provided with medium and cells, and wherein the microtiter plate is provided with a cover according to any of the claims 1 to 10, and the covered microtiter plate is shaken using a shaker.

The method also includes any modification as discussed above for the cover.

Finally the invention relates to a method of providing a cover with a filter, wherein the cover is a cover as defined in claim 9 as well as any modification thereof in the discussion of the claims related to the cover. According tot the method, the cover for a microtiter plate comprises providing a filter strip into a recess for a filter by inserting an oblong tab to which the filter strip attached into the recess at one side of the cover, passing said tab to the opposite side of said cover and pulling the filter strip into the recess for the filter, wherein the tab
- has a length greater than the distance between the two opposite sides,
- is relatively rigid compared to the filter, and
- is relatively thin with respect to the filter .

Typically the tab will be detached, e.g. by cutting the filter strip. The oblong tab is for example a strip of metal or carbon-composite to which the filter strip is attached mechanically (clamping) or with an adhesive, such as with tape.

The present invention will now be illustrated with reference to the drawings, where
Figs. 1A - Fig. 1B show a perspective view and a cut-out view of a microtiter plate provided with a cover according to the present invention respectively;
Fig. 2 shows a perspective top view of the cover of Fig. 1 to illustrate its assembly;
Figs. 3A - Fig. 3B show a perspective bottom view, and a cross-sectional view through the cover of Fig. 1 respectively;
Figs. 4A -Fig. 4B show in more detail a cross-sectional view of the cover of Fig. 1A while provided on a microtiter plate.
Figs. 5A-5B a top view and a bottom view of an elastomeric body used in the cover of Fig. 1 respectively;
Fig. 6 shows an alternative embodiment of the cover of Fig. 2 without a main body (plate);
Fig. 7 illustrates a method according to the invention and shows a top view of the cover of Fig. 6 provided with a main body (plate); and
Fig. 8 corresponds with the elastomeric body of Fig. 6, showing an alternative embodiment thereof.

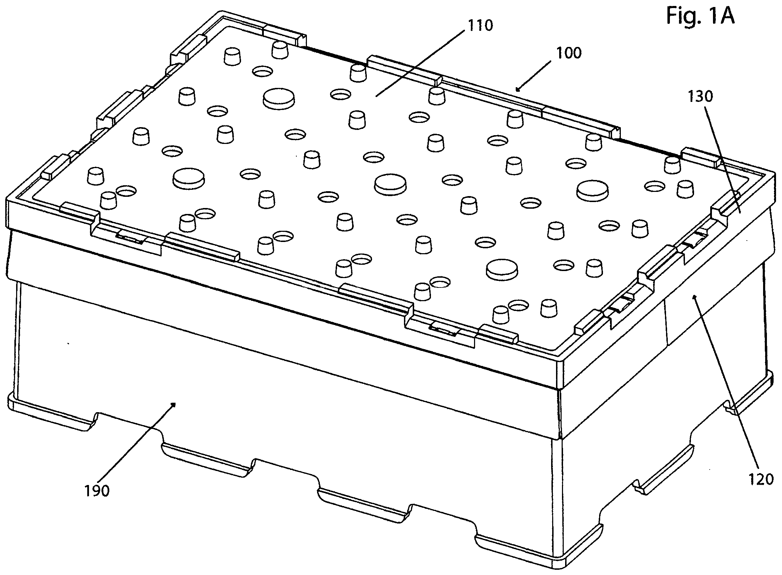

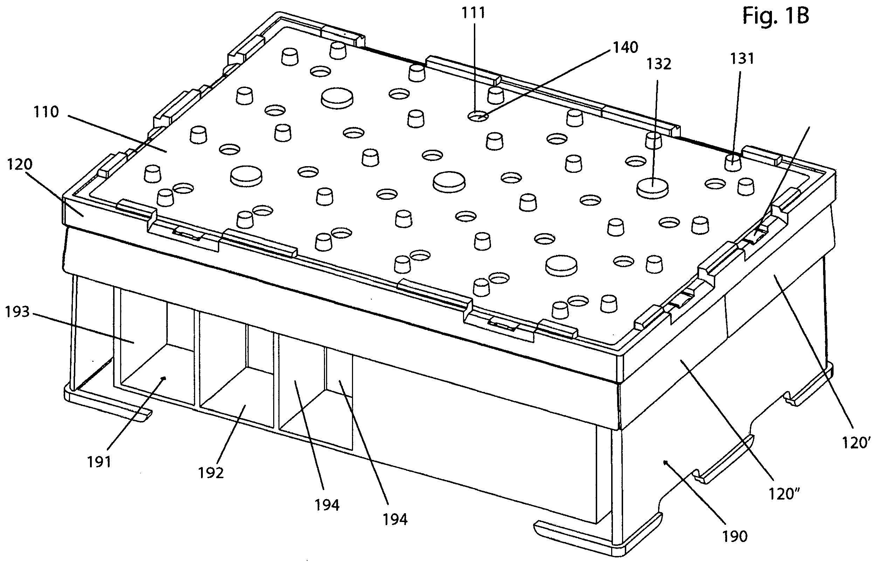

Fig. 1A shows a perspective view of a standard 24-well microtiter plate 190 (MTP) provided with a cover 100 according to the present invention. Fig. 1B corresponds to Fig. 1A and is a cut-out perspective view of said microtiter plate 190 showing square wells 191 defined by a bottom 192, upright non-partition walls 193 and upright partition walls 194. Partition walls 194 are walls that adjacent wells have in common.

The embodiment of the cover 100 discussed here comprises a lid consisting of a rectangular, flat main body (plate) 110, here a carbonfiber-resin composite plate 110 with a thickness of 1 mm. An elastomeric body 130 is fixed to the flat main body 110, as detailed below.

At the circumference of the cover 100 are members 120, here formed by two bent metal strips 120', 120" with a thickness of 0.3 mm, that together with the flat main body 110 define a space for receiving a top side of the microtiter plate 190. In the present embodiment, the metal strips 120', 120" comprise tabs 121 that are bent and received in recesses in the elastomeric body 130, as will be detailed below. To facilitate (manual or robotic) placement on an MTP, the metal strips flare out (4° to the normal of the flat main body 110).

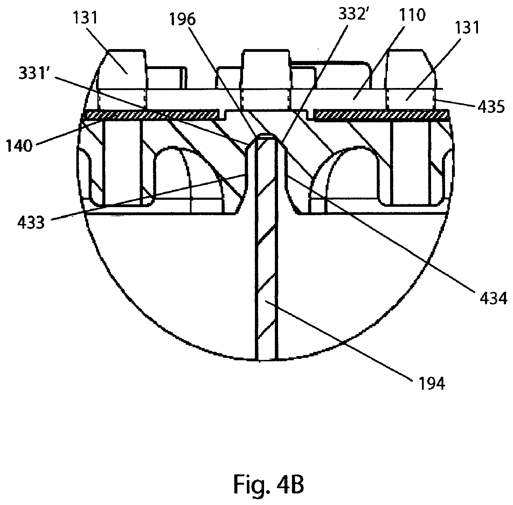

The rectangular elastomeric body 130 comprises protrusions 131 that are passed through undersized through-holes 435 (Fig. 4B) in the flat main body 110 to fix the rectangular elastomeric body to the lid and ensure that the grid of grooves remains parallel to the flat main body. There are also further protrusions 132 protruding through-holes in the flat main body 110 for supporting the bottom side of a further MTP 190 (not shown). These further protrusions 1323 are not protruding further than the protrusions 131 as the former engage relatively low parts of the bottom of said further MTP.

The flat main body 110 and the elastomeric body comprise through-holes 111, 231 for gas exchange between the wells 191 and the surrounding atmosphere via a microporous filter 140 (here four filters 140, as will be discussed below).

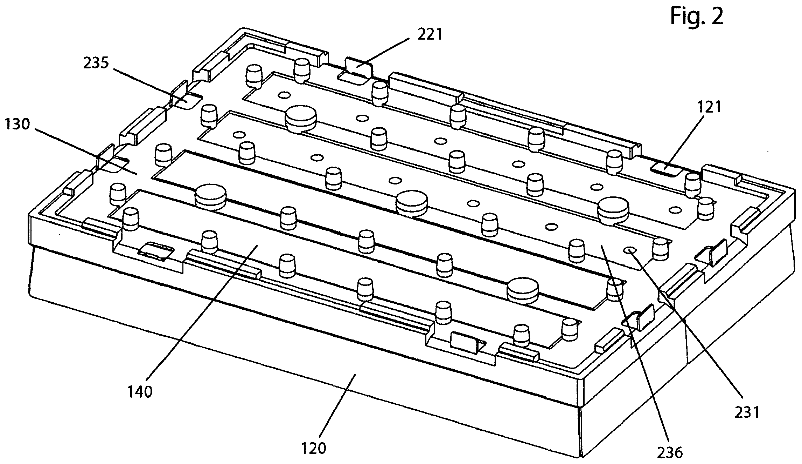

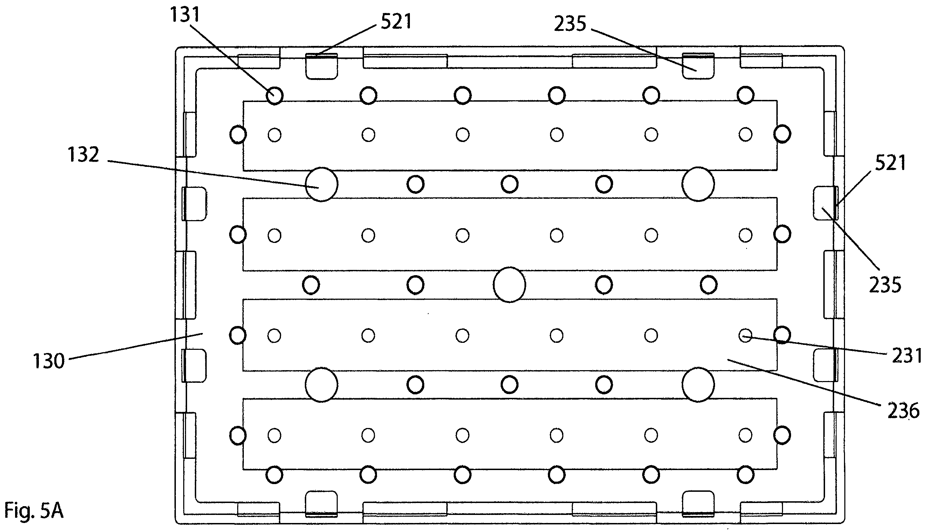

Fig. 2 shows a perspective top view of the cover 100 of Fig. 1 to illustrate its assembly. In Fig. 2 the plate 110 is not yet provided on the elastomeric body 130. It can be seen that unbent tabs 221 of the members 120 protrude through slots 521 (Fig. 5A) in the elastomeric body 130, with the tabs 121 having already been bent and received in recesses 235 in the elastomeric body 130. The cover of this embodiment comprises four filters 140 in filter recesses 236; two of the filter recesses 236 are shown provided with filters 140 while the remaining two filter recesses 236 are shown empty. There the through-holes 231 for exchange of gases through the elastomeric body 130 can be seen.

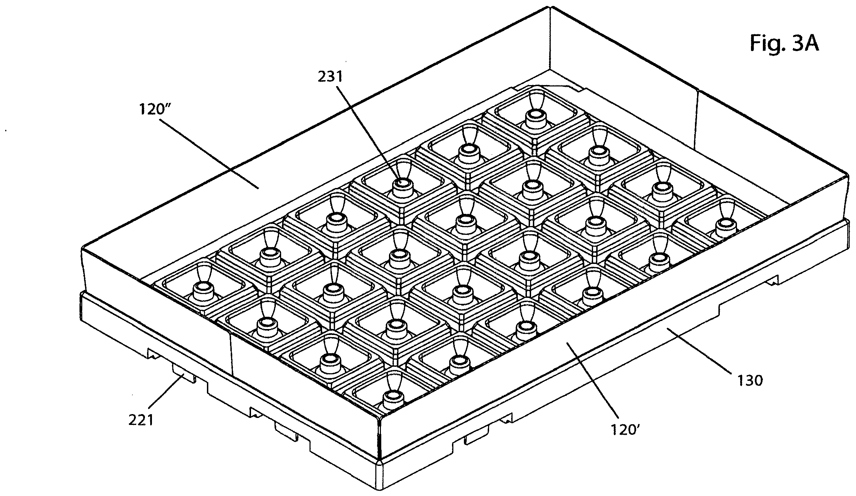

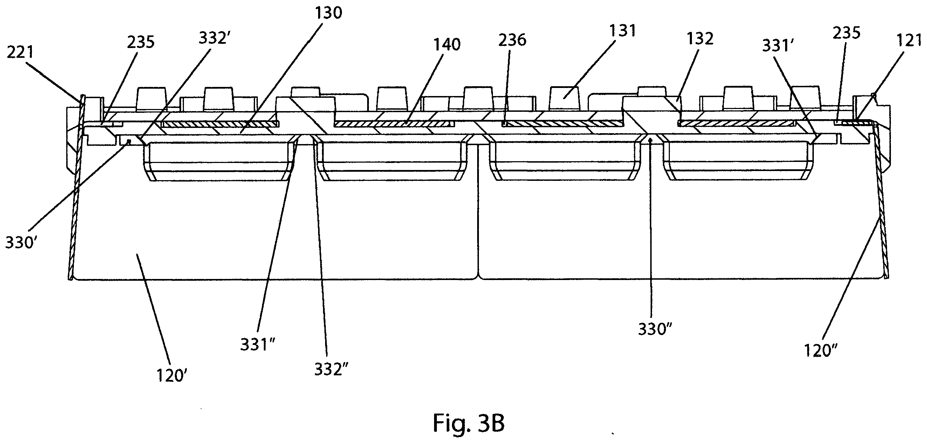

Figs. 3A shows a perspective bottom view through the cover 100 of Fig. 1. Fig. 3B shows a cross-sectional view of said cover 100 without the main body 110, with the unbent tab 221 not yet bent to be received in tab recess 235.

The elastomeric body 130 has grooves 330', 330" for receiving upright walls 193, 194 of the wells, here flat-bottomed V-shaped grooves 330" for receiving partition walls with a first groove wall section 331 at 45° and a second groove wall section 332 at 135° with respect to the flat main body 110. For non-partition walls 193, an outward facing first groove wall section 331' is present and a second groove wall section 332'.

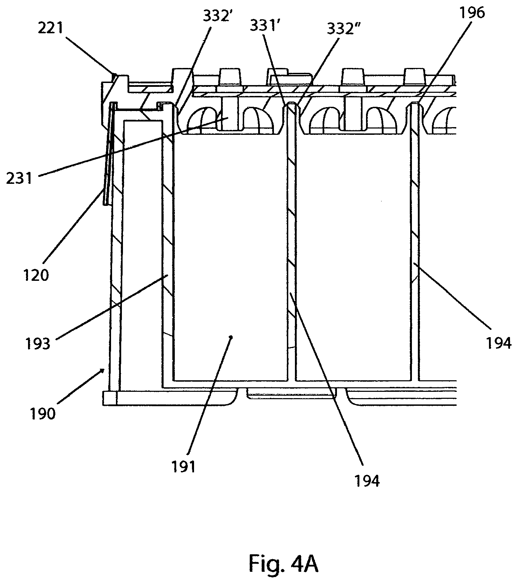

Figs. 4A shows a detail of a cross-sectional view of the cover of Fig. 1A while provided on a microtiter plate. It can be seen that edges 196 of the upper section of the upright walls 193, 194 contact the groove wall sections 331', 332' of the grooves 330.

Fig. 4B shows a third longitudinally extending groove wall section 433 at a third angle of 90° with respect to a main plane of the flat main plate 110 and a fourth longitudinally extending groove wall section 434 at a fourth angle of 90° with respect to a main plane of the flat main plate 110.

The width of a 24-well plate is typically about 1.2 mm and the third longitudinally extending groove wall section 433 and the fourth longitudinally extending groove wall section 434 spaced 2.4 mm apart helps to ensure proper sealing and avoiding contamination. The cover doesn't clamp (stick) to the MTP, allowing the cover to be lifted with, for example, a robot arm.

The groove may flare out further, to facilitate placing the cover onto an MTP.

Fig. 4B shows the flat rectangular plate 110, as well as the undersized through-holes 435 for the protrusions 131 and filters 140 sandwiched between the elastomeric body 130 and the plate 110.. Figs. 5A-5B a top view and a bottom view of an elastomeric body 130 used in the cover 100 of Fig. 1 respectively. It is made of Santoprene^{™} with a Shore A hardness of 40. Fig. 5A shows slots 521 for the tabs 121 of the members 120, and recesses 235 for said tabs 121. Fig. 5A also shows four recesses 236 for the filters 140.

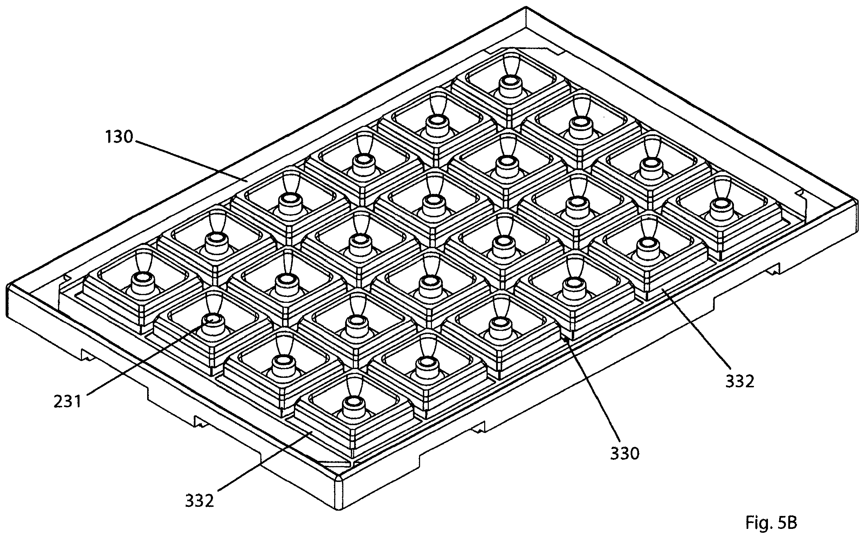

Fig. 5B shows a bottom view of the elastomeric body 130 with grooves 330 and through-holes 231 for exchange of gasses between a well and the atmosphere.

The cover of Fig. 1 as described above weighs only 68 grams (with steel lid instead of carbonfiber-epoxy composite 123 g) compared to a traditional cover of 200 grams. A 24-well MTP with medium weighs about 185 grammes. This means that the load experienced by a shaker, which may carry 12 MTPs (or more if stacked) is significantly less, allowing for less vibrations and a reduced risk of affecting the growth of cells grown in the MTPs.

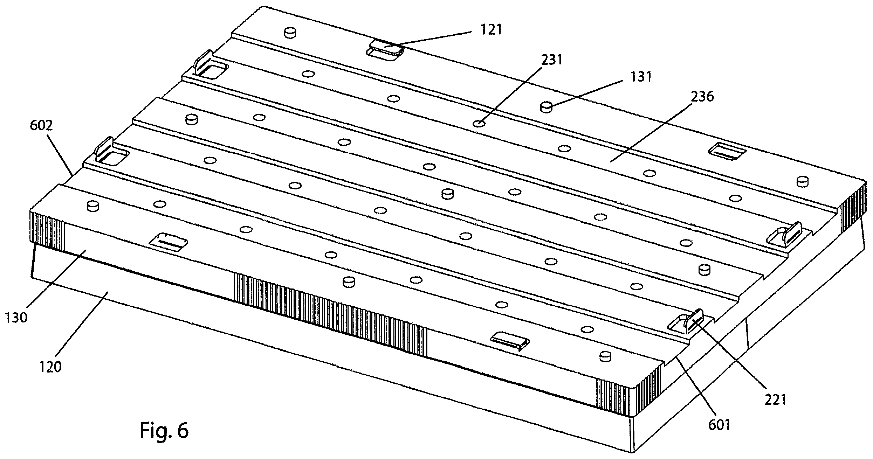

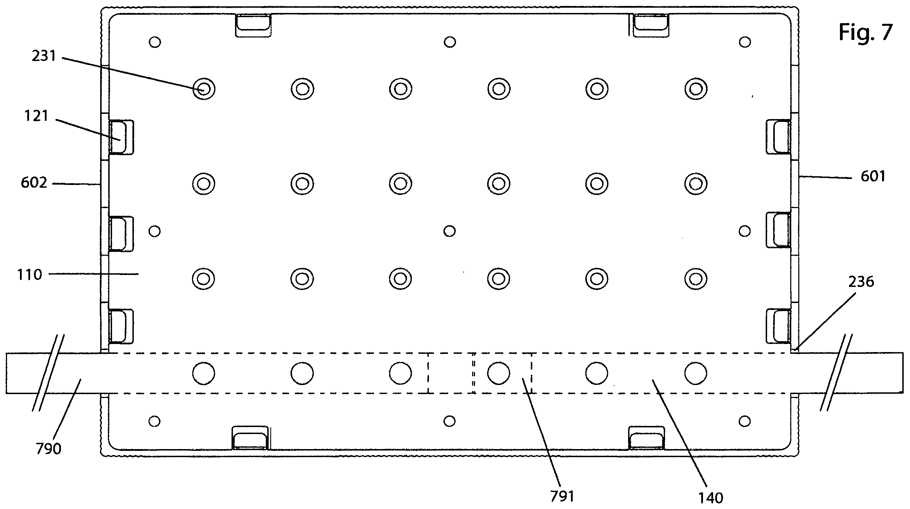

Fig. 6 shows an alternative embodiment of the cover 100 of Fig. 2 without the main body (plate) during assembly (not all unbent tabs 221 have been bent yet). More specifically it shows that the elastomeric body 130 comprises four filter recesses 236 extending from a first side 601 to an opposite second side 602. These can be used as illustrated with reference to Fig. 7 to provide a filter 140 to the cover 100 during assembly or for replacing an old filter after the cover has been used for a while. An oblong tab 790 is provided with an oblong filter 140 (filter strip 140), for example using adhesive tape 791. Here the tab is a strip of carbonfiber-reinforced composite, but it may be of plastic, cardboard or metal as desired. The tab 790 is inserted at the first side 601 into the filter recess 236 and when its free end appears at the second side 602 the tab 790 plus filter strip is pulled through the filter recess 236. With a filter strip the length of (more than) four filter recesses 236, the same tab can be used to provide each filter recess 236 with a filter strip until the free end of the filter strip is about to disappear behind the plate 110, following which the filter strip is cut at the second side 620.

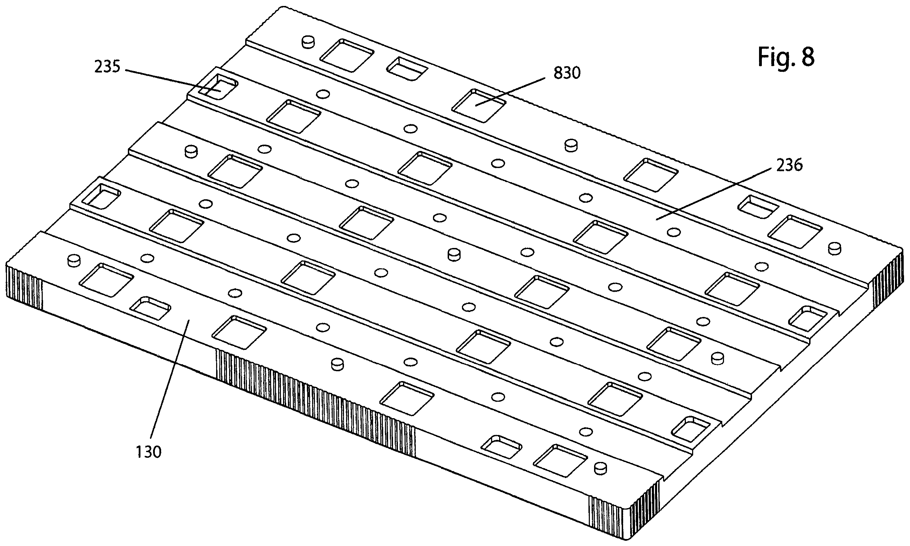

Fig. 8 corresponds with the elastomeric body 130 of Fig. 6, showing an alternative embodiment thereof. It comprises recesses (adhesive well) 830 into which an adhesive may be deposited for manufacture of the cover, said adhesive being confined within the adhesive well. Its viscosity is chosen such that upon deposition in the recess the adhesive protrudes above the recess 830 and is flattened by contact with the main body 110. The amount of adhesive is chosen to be less than the volume of the adhesive well.

In a cover according to the present invention, the elastomeric body may be bonded to the main body using double-sided adhesive tape, such as polyacrylate tape, which may be provided in adhesive wells if present.

## Claims

1. A cover (100) for a microtiter plate (190),
said microtiter plate (190) comprising a multitude of square wells (191), each well being defined by a bottom (192) and upright walls (193) chosen from partition walls (194) adjacent to other wells (191) and non-partition walls (193),
wherein the cover (100) comprises
- a lid comprising a rectangular, flat main body (110),
- at the circumference of the cover (100) members (120) that together with the flat main body (110) define a space for receiving a top side of the microtiter plate (190),
- a rectangular elastomeric body (130), and
- a microporous filter (140) in said space sandwiched between the main body (110) and the elastomeric body (130);
wherein the lid and the elastomeric body (130) comprise through-holes for gas exchange between the wells (191) and the surrounding atmosphere via the microporous filter (140);
**characterized in that** the rectangular, flat main body (110) is a plate; and the elastomeric body (130) comprises a grid of grooves (330) for receiving an upper portion of the upright walls (193) of the microtiter plate (190), grooves (330) of the grid comprising at least one of
- a first longitudinally extending groove wall section (331) at a first angle between 20° and 75° with respect to a main plane of the flat main body (110), and
- a second longitudinally extending groove wall section (332) at an angle between 160° and 105°;
wherein the groove wall sections are located for contacting said upper portion of the microtiter plate (190), with a non-partition wall (193) being in contact with a groove wall section chosen from the first and the second groove wall section (331, 332) and a partition wall (194) being in contact with both a first and a second groove wall section (331, 332) of a groove; and
wherein the elastomeric body (130) has a Shore A hardness between 20 and 90.

2. The cover (100) according to claim 1, wherein the elastomeric body (130) comprises protrusions (131) at a side of the elastomeric body (130) facing the lid and the main body (110) of the lid comprises undersized holes (435) for receiving the protrusions (131), and the elastomeric body (130) is attached to the main body (110) with the protrusions (131) received in the undersized holes (435) in the main body (110).

3. The cover (100) according to claim 2, wherein the elastomeric body (130) comprises further protrusions (132) at a side of the elastomeric body (130) facing the lid and the flat main body (110) of the lid comprises holes for receiving the further protrusions (132), and with the elastomeric body (130) attached to the main body (110) with the protrusions (131) received in the undersized holes in the main body (110) the further protrusions (132) protrude from the flat main body (110).

4. The cover (100) according to any of the preceding claims, wherein the members (120) at the circumference of the cover (100) comprise a metal strip comprising a strip body with a multitude of tabs (121), said tabs (121) extending through slots (521) in the elastomeric body (130) with the distal end of the tabs (121) transverse to the strip body.

5. The cover (100) according to any of the preceding claims, wherein the grooves (330) of the grid comprise at least one of
- a third longitudinally extending groove wall section (433) at a third angle between 70° and 90° with respect to a main plane of the flat main body (110) and with the first longitudinally extending groove wall section (433) between a bottom (192) of the groove and the third longitudinally extending groove wall section (433), and
- a fourth longitudinally extending groove wall section (434) at a fourth angle between 90° and 110° with respect to a main plane of the flat main body (110) and with the third longitudinally extending groove wall section (433) between a bottom (192) of the groove and the fourth longitudinally extending groove wall section (434).

6. The cover (100) according to any of the preceding claims, wherein the flat main body (110) is a resilient metal plate.

7. The cover (100) according to any of the claims 1 to 5, wherein the flat main body (110) is a carbonfiber-resin composite plate (110).

8. The cover (100) according to any of the preceding claims, wherein at least one of the flat main body (110) and the elastomeric body (130) comprises at least one recess (236) for the filter.

9. The cover (100) according to claim 8, wherein the at least one recess (236) for a filter extends from one side of the cover (100) to an opposite side of said side.

10. The cover (100) according to any of the preceding claims, wherein at least one of the flat main body (110) and the elastomeric body (130) comprises at plurality of adhesive wells (830) for an adhesive providing a bond between the flat main body (110) and the elastomeric body (130), said adhesive wells (830) not facing a recess (236) for the filter.

11. A method of growing cells in a microtiter plate (190) comprising square wells (191),
wherein wells (191) of the microtiter plate (190) are provided with medium and cells, and wherein the microtiter plate (190) is provided with a cover (100) according to any of the claims 1 to 10, and the covered microtiter plate (190) is shaken using a shaker.

## Patentansprüche

1. Abdeckung (100) für eine Mikrotiterplatte (190),
wobei die Mikrotiterplatte (190) eine Mehrzahl von quadratischen Vertiefungen (191) umfasst, wobei jede Vertiefung durch einen Boden (192) und aufrechte Wände (193) definiert ist, die aus Trennwänden (194) benachbart zu anderen Vertiefungen (191) und Nichttrennwänden (193) gewählt sind,
wobei die Abdeckung (100) Folgendes umfasst
- einen Deckel, der einen rechteckigen, flachen Hauptkörper (110) umfasst,
- an dem Umfang der Abdeckung (100) Elemente (120), die zusammen mit dem flachen Hauptkörper (110) einen Raum zum Aufnehmen einer Oberseite der Mikrotiterplatte (190) definieren,
- einen rechteckigen elastomeren Körper (130), und
- einen mikroporösen Filter (140) in dem Raum, der zwischen dem Hauptkörper (110) und dem elastomeren Körper (130) eingefügt ist;
wobei der Deckel und der elastomere Körper (130) Durchgangslöcher für Gasaustausch zwischen den Vertiefungen (191) und der umgebenden Atmosphäre über den mikroporösen Filter (140) umfassen;
**dadurch gekennzeichnet, dass** der rechteckige, flache Hauptkörper (110) eine Platte ist; und der elastomere Körper (130) ein Raster von Nuten (330) zum Aufnehmen eines oberen Abschnittes der aufrechten Wände (193) der Mikrotiterplatte (190) umfasst, wobei Nuten (330) des Rasters zumindest eines von Folgendem umfassen
- einem ersten sich längs erstreckenden Nutenwandteil (331) in einem ersten Winkel zwischen 20° und 75° in Bezug auf eine Hauptebene des flachen Hauptkörpers (110), und
- einem zweiten sich längs erstreckenden Nutenwandteil (332) in einem Winkel zwischen 160° und 105°; wobei die Nutenwandteile zum Kontaktieren des oberen Abschnittes der Mikrotiterplatte (190) angeordnet sind, wobei eine Nichttrennwand (193) in Kontakt mit einem Nutenwandteil ist, der aus dem ersten und dem zweiten Nutenwandteil (331, 332) gewählt ist, und eine Trennwand (194) in Kontakt mit sowohl einem ersten als auch einem zweiten Nutenwandteil (331, 332) einer Nut ist; und wobei der elastomere Körper (130) eine Shore-A-Härte zwischen 20 und 90 aufweist.

2. Abdeckung (100) nach Anspruch 1, wobei der elastomere Körper (130) Vorsprünge (131) an einer Seite des elastomeren Körpers (130) umfasst, die dem Deckel zugewandt ist, und der Hauptkörper (110) des Deckels unterdimensionierte Löcher (435) zum Aufnehmen der Vorsprünge (131) umfasst und der elastomere Körper (130) an dem Hauptkörper (110) angebracht ist, wobei die Vorsprünge (131) in den unterdimensionierten Löchern (435) in dem Hauptkörper (110) aufgenommen sind.

3. Abdeckung (100) nach Anspruch 2, wobei der elastomere Körper (130) weitere Vorsprünge (132) an einer Seite des elastomeren Körpers (130) umfasst, die dem Deckel zugewandt ist, und der flache Hauptkörper (110) des Deckels Löcher zum Aufnehmen der weiteren Vorsprünge (132) umfasst, und wobei der elastomere Körper (130) an dem Hauptkörper (110) angebracht ist, wobei die Vorsprünge (131) in den unterdimensionierten Löchern in dem Hauptkörper (110) aufgenommen sind, wobei die weiteren Vorsprünge (132) von dem flachen Hauptkörper (110) vorspringen.

4. Abdeckung (100) nach einem der vorhergehenden Ansprüche, wobei die Elemente (120) an dem Umfang der Abdeckung (100) einen Metallstreifen umfassen, der einen Streifenkörper mit einer Mehrzahl von Laschen (121) umfasst, wobei sich die Laschen (121) durch Schlitze (521) in dem elastomeren Körper (130) erstrecken, wobei das distale Ende der Laschen (121) quer zu dem Streifenkörper ist.

5. Abdeckung (100) nach einem der vorhergehenden Ansprüche, wobei die Nuten (330) des Rasters zumindest eines von Folgendem umfassen
- einem dritten sich längs erstreckenden Nutenwandteil (433) in einem dritten Winkel zwischen 70° und 90° in Bezug auf eine Hauptebene des flachen Hauptkörpers (110) und mit dem ersten sich längs erstreckenden Nutenwandteil (433) zwischen einem Boden (192) der Nut und dem dritten sich längs erstreckenden Nutenwandteil (433), und
- einem vierten sich längs erstreckenden Nutenwandteil (434) in einem vierten Winkel zwischen 90° und 110° in Bezug auf eine Hauptebene des flachen Hauptkörpers (110) und mit dem dritten sich längs erstreckenden Nutenwandteil (433) zwischen einem Boden (192) der Nut und dem vierten sich längs erstreckenden Nutenwandteil (434).

6. Abdeckung (100) nach einem der vorhergehenden Ansprüche, wobei der flache Hauptkörper (110) eine elastische Metallplatte ist.

7. Abdeckung (100) nach einem der Ansprüche 1 bis 5, wobei der flache Hauptkörper (110) eine Kohlefaser-Harz-Verbundplatte (110) ist.

8. Abdeckung (100) nach einem der vorhergehenden Ansprüche, wobei zumindest einer von dem flachen Hauptkörper (110) und dem elastomeren Körper (130) zumindest eine Ausnehmung (236) für den Filter umfasst.

9. Abdeckung (100) nach Anspruch 8, wobei sich die zumindest eine Ausnehmung (236) für einen Filter von einer Seite der Abdeckung (100) zu einer gegenüberliegenden Seite der Seite erstreckt.

10. Abdeckung (100) nach einem der vorhergehenden Ansprüche, wobei zumindest einer von dem flachen Hauptkörper (110) und dem elastomeren Körper (130) eine Vielzahl von Klebstoffvertiefungen (830) für einen Klebstoff umfasst, der eine Bindung zwischen dem flachen Hauptkörper (110) und dem elastomeren Körper (130) bereitstellt, wobei die Klebstoffvertiefungen (830) nicht einer Ausnehmung (236) für den Filter zugewandt sind.

11. Verfahren zum Züchten von Zellen in einer Mikrotiterplatte (190), die quadratische Vertiefungen (191) umfasst, wobei Vertiefungen (191) der Mikrotiterplatte (190) mit Medium und Zellen bereitgestellt sind und wobei die Mikrotiterplatte (190) mit einer Abdeckung (100) nach einem der Ansprüche 1 bis 10 bereitgestellt ist und die abgedeckte Mikrotiterplatte (190) unter Verwendung eines Schüttlers geschüttelt wird.

## Revendications

1. Couvercle (100) pour une plaque de microtitrage (190),
ladite plaque de microtitrage (190) comprenant une multitude de puits carrés (191), chaque puits étant défini par un fond (192) et des parois verticales (193) choisies parmi des parois de séparation (194) adjacentes à d'autres puits (191) et des parois de non séparation (193),
ledit couvercle (100) comprenant
- un couvercle comprenant un corps principal plat rectangulaire (110),
- au niveau de la circonférence du couvercle (100), des éléments (120) qui, ensemble avec le corps principal plat (110), définissent un espace destiné à recevoir une face supérieure de la plaque de microtitrage (190),
- un corps en élastomère rectangulaire (130), et
- un filtre microporeux (140) dans ledit espace intercalé entre le corps principal (110) et le corps en élastomère (130) ;
ledit couvercle et ledit corps en élastomère (130) comprenant des trous traversants pour l'échange de gaz entre les puits (191) et l'atmosphère environnante par l'intermédiaire du filtre microporeux (140) ; **caractérisé en ce que** le corps principal plat rectangulaire (110) est une plaque ; et ledit corps en élastomère (130) comprenant une grille de rainures (330) destinée à recevoir une partie supérieure des parois verticales (193) de la plaque de microtitrage (190), les rainures (330) de la grille comprenant au moins une parmi
- une première section de paroi de rainure s'étendant longitudinalement (331) selon un premier angle compris entre 20° et 75° par rapport à un plan principal du corps principal plat (110), et
- une deuxième section de paroi de rainure s'étendant longitudinalement (332) à un angle compris entre 160° et 105° ; lesdites sections de paroi de rainure étant situées pour entrer en contact avec ladite partie supérieure de la plaque de microtitrage (190), une paroi de non séparation (193) étant en contact avec une section de paroi de rainure choisie parmi la première et la deuxième section de paroi de rainure (331, 332) et une paroi de séparation (194) étant en contact avec à la fois une première et une deuxième section de paroi de rainure (331, 332) d'une rainure ; et ledit corps en élastomère (130) possédant une dureté Shore A comprise entre 20 et 90.

2. Couvercle (100) selon la revendication 1, ledit corps en élastomère (130) comprenant des saillies (131) sur un côté du corps en élastomère (130) faisant face au couvercle et le corps principal (110) du couvercle comprenant des trous sous-dimensionnés (435) destinés à recevoir les saillies (131), et ledit corps en élastomère (130) étant fixé au corps principal (110) avec les saillies (131) reçues dans les trous sous-dimensionnés (435) dans le corps principal (110).

3. Couvercle (100) selon la revendication 2, ledit corps en élastomère (130) comprenant des saillies supplémentaires (132) sur un côté du corps en élastomère (130) faisant face au couvercle et ledit corps principal plat (110) du couvercle comprenant des trous destinés à recevoir les saillies supplémentaires (132), et avec le corps en élastomère (130) fixé au corps principal (110) avec les saillies (131) reçues dans les trous sous-dimensionnés dans le corps principal (110), lesdites saillies supplémentaires (132) faisant saillie à partir du corps principal plat (110).

4. Couvercle (100) selon l'une quelconque des revendications précédentes, lesdits éléments (120) au niveau de la circonférence du couvercle (100) comprenant une bande métallique comprenant un corps de bande avec une multitude de pattes (121), lesdites pattes (121) s'étendant à travers des fentes (521) dans le corps en élastomère (130) avec l'extrémité distale des pattes (121) transversale au corps de bande.

5. Couvercle (100) selon l'une quelconque des revendications précédentes, lesdites rainures (330) de la grille comprennent au moins une parmi
- une troisième section de paroi de rainure s'étendant longitudinalement (433) selon un troisième angle compris entre 70° et 90° par rapport à un plan principal du corps principal plat (110) et la première section de paroi de rainure s'étendant longitudinalement (433) entre un fond (192) de la rainure et la troisième section de paroi de rainure s'étendant longitudinalement (433), et
- une quatrième section de paroi de rainure s'étendant longitudinalement (434) selon un quatrième angle compris entre 90° et 110° par rapport à un plan principal du corps principal plat (110) et avec la troisième section de paroi de rainure s'étendant longitudinalement (433) entre un fond (192) de la rainure et la quatrième section de paroi de rainure s'étendant longitudinalement (434).

6. Couvercle (100) selon l'une quelconque des revendications précédentes, ledit corps principal plat (110) étant une plaque métallique élastique.

7. Couvercle (100) selon l'une quelconque des revendications 1 à 5, ledit corps principal plat (110) étant une plaque composite en fibre de carbone-résine (110).

8. Couvercle (100) selon l'une quelconque des revendications précédentes, au moins un parmi le corps principal plat (110) et le corps en élastomère (130) comprenant au moins un évidement (236) pour le filtre.

9. Couvercle (100) selon la revendication 8, ledit au moins un évidement (236) pour un filtre s'étendant à partir d'un côté du couvercle (100) jusqu'à un côté opposé dudit côté.

10. Couvercle (100) selon l'une quelconque des revendications précédentes, au moins un dudit corps principal plat (110) et dudit corps en élastomère (130) comprenant une pluralité de puits adhésifs (830) pour un adhésif fournissant une liaison entre le corps principal plat (110) et le corps en élastomère (130), lesdits puits adhésifs (830) ne faisant pas face à un évidement (236) pour le filtre.

11. Procédé de culture de cellules dans une plaque de microtitrage (190) comprenant des puits carrés (191), des puits (191) de la plaque de microtitrage (190) étant pourvus d'un milieu et de cellules, et ladite plaque de microtitrage(190) étant pourvue d'un couvercle (100) selon l'une quelconque des revendications 1 à 10, et ladite plaque de microtitrage recouverte (190) étant agitée à l'aide d'un agitateur.
